# EUROPEAN PATENT APPLICATION

(11) **EP 0 611 013 A1**
(43) Date of publication of application: **17.08.1994**
(21) Application number: 94200801.2
(22) Date of filing: 24.08.1989
(51) Int. Cl.: A61M 25/06, A61M 25/01

(54) **Femoral arterial cannula**

(30) Priority: 29.08.1988 US 238154
(62) Divisional of application: 89308580.3
(71) Applicant: SORIN BIOMEDICAL INC., Irvine, California 92713-9503 (US)
(72) Inventor: Buckberg, Gerald D., Los Angeles, California (US); Jones, Kenneth A., Lake Elsinore, California (US); Maloney, James V., Jr., Los Angeles, California (US); West, Weldon D., Mission Viejo, California (US)
(74) Representative: Smulders, Theodorus A.H.J., Ir.

(57) **Abstract**

A cannula assembly (65) comprising an elongated tubular cannula body (72) having a tip section (72a) and a stylet (66) slidebly positioned within the cannula body such that one end of the stylet can extend beyond said tip section. The stylet includes a stop (66b) on its outer periphery which slides within the interior of the cannula body and limits withdrawal of the stylet when engaging an anti-backflow ring (80) in the end of the cannula body remote from the tip section (72a) to prevent leakage out of the cannula body.Preferably, the ring (80) can be forced out of the cannula body and a clamp (71) is present for limiting fluid flow into the cannula body.

## Description

The invention relates to a cannula assembly comprising an elongated tubular cannula body having a tip section and a stylet slidably positioned within the cannula body such that one end of the stylet can extend beyond said tip section.

A cannula assembly of said type is known from US-A-4,511,356 and is meant for use in operations such as lumbar disc puncture. Means are present to temporarily lock the stylet relative to the cannula body when the point of the stylet extends slightly beyond the tip section. After disengaging the stylet from the cannula body the stylet can be completely pulled out of the cannula body, e.g. to be replaced by another instrument.

In recent years, there has been developed a femoral-to-femoral cardiopulmonary bypass system wherein it is not necessary to open the patient's chest. To accomplish this, it is necessary to employ large diameter cannulas and catheters in order to maximize blood flow. Cannulating a femoral artery is particularly difficult because of the pressure involved as well as the large flow through the artery.

A cannula of the said last mentioned type is known from EP-A-0 232 074. Preparatory to insertion of this cannula, an incision has to be made being large enough to bring a flat shoulder in contact with the vascular conduit; all the more so, because there should be room for rotating the cannula around. In other words a stylet for smoothly penetrating a femoral artery is not present.

The object of the invention is to provide an improved cannula which can be inserted into a femoral artery with a minimum of blood loss, in a minimum of time, and with a minimum of trauma and risk to the patient.

This object is according to the invention achieved in that the stylet includes a stop on its outer periphery which slides within the interior of the cannula body and limits withdrawal of the stylet when engaging a seal in the end of the cannula body remote from the tip section to prevent leakage out of the cannula body. In use, the stylet is placed in the position with its one end extending beyond the tip section of the cannula body. After introducing the tip section into the femoral artery the seal, e.g. an anti-backflow ring positioned in the end of the cannula body remote from the tip section, prevents leakage through the annular passage between the stylet and the cannula body.

When it is desirable to connect the cannula assembly to an extracorporeal circuit, it is preferred that the anti-backflow ring forms a temporary limit for said stop when said stylet is in the process of being withdrawn from the cannula body. In that case the stylet is preferably adapted to be fully withdrawn from said cannula body including forcing said ring out of the cannula body, whereas the stylet and the cannula body have such lengths that when said stop engages the ring to temporary limiting withdrawal of the stylet, the cannula body extends beyond the one end of the stylet, and means, e.g. a clamp for squeezing the cannula body, are present at a location between the one end of the stylet engaging the ring and the tip of the cannula body for limiting fluid flow into the cannula body. Then, connecting the cannula assembly with e.g. a pump will be possible nearly without loss of blood by withdrawing the stylet into contact with the anti-backflow ring, squeezing the clamp and completely withdrawing the stylet. At that moment only a small unpressured quantity of blood is present in the open end of the cannula body, so that nearly without loss of blood the desired connections can be made.

The objects and advantages of the invention will become more apparent from the following description taken in conjunction with the accompanying drawings, wherein:
Figure 1 is a schematic perspective view of an arterial cannula inserted in a femoral artery;
Figure 2 is a view similar to Figure 1, but with the stylet of the cannula assembly partially withdrawn;
Figure 3 is a perspective view of the cannula assembly of Figures 1 and 2;
Figure 4 is a perspective view of the stylet of the assembly of Figure 3;
Figure 5 is a perspective view of an anti-backflow ring of the cannula assembly;
Figure 6 is an enlarged perspective schematic view of the cannula assembly inserted in a femoral artery;
Figure 7 is a side elevational, partially sectionalized view of the assembly of Figure 3;
Figure 8 is a cross-sectional view of the cannula assembly with the stylet partially withdrawn and with the cannula clamped to prevent flow therethrough:
Figure 9 is a cross-sectional view on line 9-9 of Figure 7.

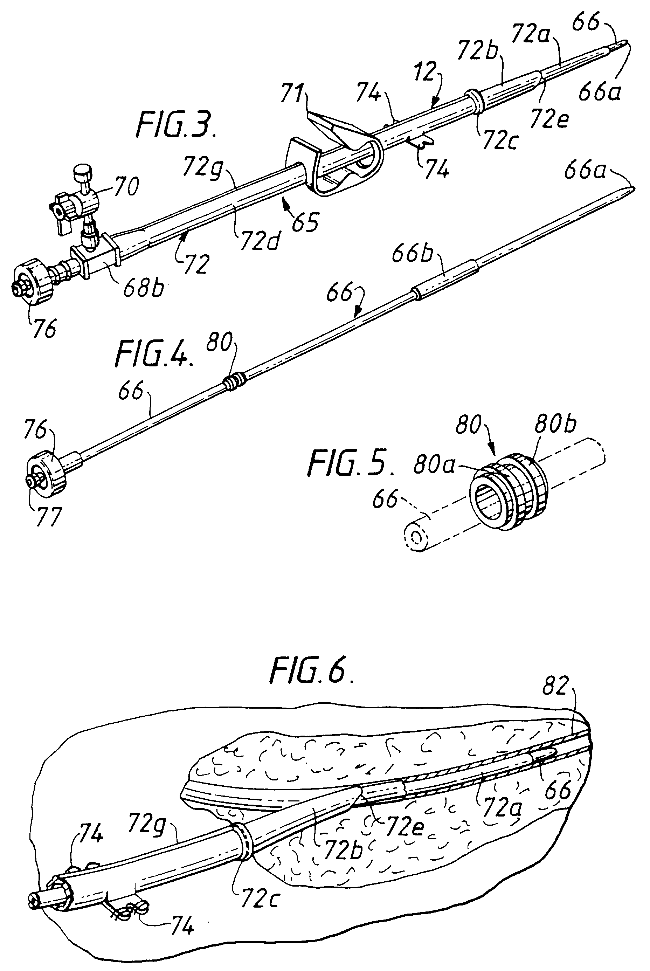

A femoral arterial cannula assembly 65 is shown in Figure 3. The assembly includes an elongated tubular body 72, a stylet 66 which is slidably received within the cannula body, a fitting 68 secured to the proximal end of the cannula body, a stopcock 70 on the fitting, and a clamp 71.

The cannula body 72 is preferably made from a medical-grade polyvinyl chloride, or silicone rubber. The body 72 has three primary sections including a distal tip section 72a for insertion into the femoral artery, an intermediate section 72b adapted to extend from the artery to a raised suture stabilizing ring 72c to be located near the exterior skin of the leg, and an exterior section 72d extending from the stabilizing ring 72c to the fitting 68.

The tip of section 72a is tapered to facilitate insertion into an artery. In a preproduction version of the cannula body, the maximum exterior diameter of the tip section 72a is 6.68 mm and the maximum interior diameter is 5.08 mm. The intravascular length of the tip section 72a in a preproduction version is 5.88 cm, which is a desirable length to ensure adequate seating within the artery.

The tapering intermediate section 72b has a length of about 5 cm, with a maximum interior diameter of about 9.27 mm, and with an exterior diameter which tapers from the tip end of about 8.4 mm and increases to about 1.2-1.3 cm. Thus, it can be seen that there is a significant exterior diameter change from the tip section 72a to the tip end of the intermediate section 72b. This diameter change forms an annular shoulder 72e adapted to engage the exterior of an artery. That shoulder is formed at an angle of approximately 45° with respect to a diametrical plane through the cannula body. It has been found that this angle is particularly desirable for sealing with the exterior of the femoral artery when the cannula body is inserted in the direction towards the heart.

Due to the angled shoulder and the angle at which the tip section is inserted in the femoral artery, it is important the cannula body be rotationally oriented properly. Thus, for orientation purposes, there is formed an elongated orientation rib 72g on the exterior of sections 72b and 72d extending from the stabilizing ring 72c to the proximal end of the body which mates with the fitting 68. The section 72d is about 19 cm in length, making the overall length of the cannula body about 33 cm. The interior and exterior diameters are constant with the end of section 72b.

A pair of suture wings or flaps 74 are formed integral with and extend outwardly from the exterior section 72d. The flaps form a lower surface which is approximately tangent with the exterior of the section 72d. The suture wings in a prototype are positioned about 5 cm from the stabilizing ring 72c.

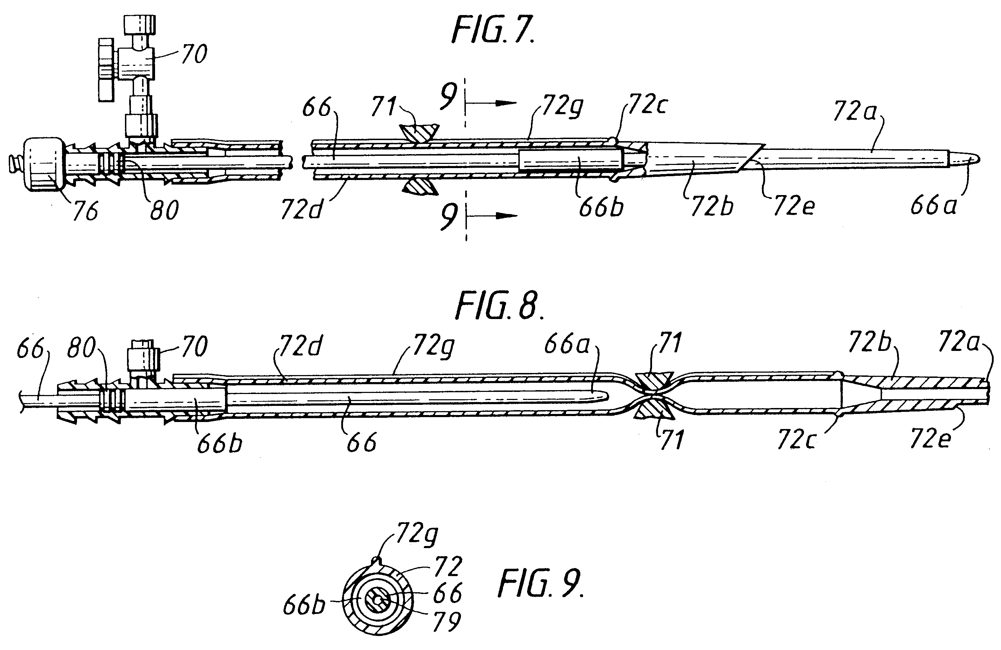

Referring to Figures 3 and 4, the stylet 66 is an elongated flexible tubular element preferably made of medical-grade polyvinyl chloride or silicone rubber. The stylet 66 is slightly longer than the cannula body 72 having a tapered tip 66a on one end which extends beyond the tip of the cannula body when the stylet is fully inserted therein. The overall length is about 42 cm. A knob 76 on the other end of the stylet extends beyond the fitting 68 and is useful for installing and removing the stylet from the cannula body. The diameter of the stylet is slightly smaller than the inner diameter of the tip section. A small diameter lumen 77 is formed throughout the length of the catheter and is adapted to receive a small diameter guidewire. Included on the stylet 66 is an elongated cylindrical stop 66b having an exterior diameter which is sized to slide relatively easily within the cannula body, but nevertheless there is resistance to movement caused by the stop 66b engaging the interior of the cannula body. The stop 66b is spaced from the tip end of the stylet about 15 cm and has a length of about 4 cm. This means that the end of the stop 66b closest to the tip 66a of the stylet is located at the stabilizing ring of the cannula body when the stylet is fully inserted in the cannula body, as seen in Figure 7.

Also included in the cannula assembly 65 is an anti-backflow ring 80 preferably made of medical-grade silicone rubber. The ring has a short barrel-like tubular shape with two axially spaced outwardly extending annular ribs 80a. The ribs are dimensioned such that the backflow ring fits fairly tightly within the interior of the fitting 68 or the cannula body; and in that position, fits snugly on the stylet 66.

Mounted on the exterior of the cannula body between the suture wings and the fitting 68 is the adjustable medical clamp 71. The clamp is of standard construction, adapted to be manually set to pinch the cannula body closed when the stylet is removed.

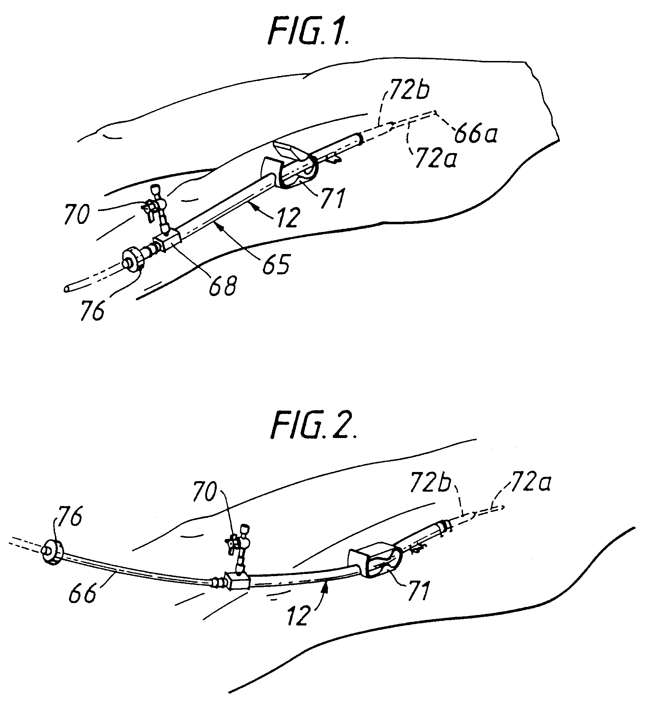

In use, the guidewire is inserted into the femoral artery followed by the cannula body with the stylet fully inserted, as shown in Figures 1, 3 and 6. The cannula body is preferably introduced by use of the Seldinger technique, or a cutdown procedure along with vessel dilation. The assembly, guided by the guidewire, is inserted to the point where the angled shoulder 72e engages the exterior of an artery 82 with the tip section 72a extending into the artery, together with the tip of the stylet, as illustrated in Figure 6. It can be seen from that figure that the angled shoulder 72e seal engages the artery in a manner such that the artery substantially conforms to the shoulder with the exterior of the artery, and limits insertion. With the cannula body so positioned, it is sutured to the leg by means of the wings, and the raised suture ridge, as shown in Figure 6.

With the cannula body so positioned, there is little blood leakage through the hole in the artery as a result of the seal with the shoulder 72e. Also, the ring 80 prevents leakage through the annular passage between the stylet and the cannula body, as seen in Figure 7. With the forward portion of the cannula body relatively firmly positioned on the patient's leg, the proximal end of the cannula body is free to be raised or manipulated, as may be appreciated from Figure 2.

When it is desirable to connect the cannula body to an extracorporeal circuit, the stylet 66 is partially withdrawn by pulling on the knob 76 to the position shown in Figure 8, wherein the stop 66b on the stylet engages anti-backflow ring 80 in the fitting. Note that there is firm resistance by the ring to withdrawing the ring 80 out of the cannula body so that an operator recognizes the stopping point. The tip 66a of the stylet is spaced considerably from the stabilizing ring since the length of the stylet from the stop 66b to its tip 66a is somewhat less than the length of the cannula body from its fitting end to the stabilizing ring 72c. With the stylet 66 so positioned, the clamp 71 is squeezed onto the exterior section 72d at a location between the tip 66a of the stylet and the stabilizing ring 72c, as seen in Figure 8. By locating the clamp close to the tip 66a of the stylet, there is only a small quantity of blood between the clamp and the ring 80. Once the clamp has been positioned and closed, the stylet can be withdrawn completely with a pulling force greater than that to move the stop, such that the ring 80 in the end of the fitting is also withdrawn, captured on the stylet 66. At this stage, there is only a small quantity of blood in the open end of the cannula body, and it is not under pressure. Because of this and the suture wings, the proximal end of the cannula body can be easily handled for making connections to a pump and priming the cannula body without loss of blood.

## Claims

1. A cannula assembly (65) comprising an elongated tubular cannula body (72) having a tip section (72a) and a stylet (66) slidably positioned within the cannula body such that one end of the stylet can extend beyond said tip section, characterized in that the stylet includes a stop (66b) on its outer periphery which slides within the interior of the cannula body and limits withdrawal of the stylet when engaging a seal (80) in the end of the cannula body remote from the tip section (72a) to prevent leakage out of the cannula body.

2. A cannula assembly (65) according to claim 1, characterized in that said seal is an anti-backflow ring (80) positioned in the end of the cannula body (72) remote from the tip section (72a) and forming a temporary limit for said stop (66b) when said stylet (66) is in the process of being withdrawn from the cannula body.

3. A cannula assembly (65) according to claim 2, characterized in that said stylet (66) is adapted to be fully withdrawn from said cannula body (72) including forcing said ring (80) out of the cannula body, the stylet and the cannula body having such lengths that when said stop (66b) engages the ring to temporary limiting withdrawal of the stylet, the cannula body extends beyond the one end of the stylet, means (71) being present at a location between the one end of the stylet engaging the ring and the tip of the cannula body for limiting fluid flow into the cannula body.

4. A cannula assembly (65) according to claim 3, characterized in that said means for limiting fluid flow into said cannula body (72) include a clamp (71) for squeezing the cannula body.

5. A cannula assembly (65) according to one of the preceding claims, characterized in that said end of the cannula body (72) remote from the tip section (72a) is provided with means for connecting said end to a pump.
